# EUROPEAN PATENT SPECIFICATION

(11) **EP 1 881 324 B1**
(45) Date of publication and mention of the grant of the patent: **21.01.2015**
(21) Application number: 07021724.5
(22) Date of filing: 10.09.2001
(51) Int. Cl.: G01N 33/542, G01N 33/569

(54) **Fluorescence polarization-based homogeneous assay for deoxynivalenol determination in grains**
Polarisationsbasierter, homogener Fluoreszenz-Assay zur Bestimmung von Deoxynivalenol in Körnern
Analyse homogène à base de polarisation fluorescente pour détermination désoxynivalénol en grains

(30) Priority: 11.09.2000 US 231887 P; 11.07.2001 US 903061
(43) Date of publication of application: 23.01.2008
(62) Divisional of application: 01975741.8
(73) Proprietor: Diachemix LLC, Milwaukee, WI 53202 (US)
(72) Inventor: Nasir, Mohammad Sarwar, Grayslake, IL 60030 (US); Jolley, Michael E., Round Lake, IL 60073 (US)
(74) Representative: Schnappauf, Georg

(56) References cited:
- DE-A1- 4 013 004
- NIELSEN K ET AL: "A homogeneous fluorescence polarization assay for detection of antibody to Brucella abortus" 9 September 1996 (1996-09-09), JOURNAL OF IMMUNOLOGICAL METHODS, ELSEVIER SCIENCE PUBLISHERS B.V.,AMSTERDAM, NL, PAGE(S) 161 - 168 , XP004021266 ISSN: 0022-1759 * page 162, column 2, paragraph 1 * * page 166, column 1, paragraph 2 * * page 167, column 1, paragraph 3 *
- LIGLER F S ET AL: "A HOMOGENEOUS IMMUNOASSAY FOR THE MYCOTOXIN T-2 UTILIZING LIPOSOMES MONOCLONAL ANTIBODIES AND COMPLEMENT" 1 January 1987 (1987-01-01), ANALYTICAL BIOCHEMISTRY, ACADEMIC PRESS INC. NEW YORK, PAGE(S) 369 - 375 , XP001062106 ISSN: 0003-2697 * page 369 * * page 374, column 2 *
- ADAMCZYK M ET AL: "SYNTHESIS OF CONJUGATES FOR A BARBITURATE SCREENING ASSAY" BIOCONJUGATE CHEMISTRY, ACS, WASHINGTON, DC, US, vol. 8, no. 3, 1 January 1997 (1997-01-01), pages 281-288, XP002085589 ISSN: 1043-1802

## Description

### BACKGROUND OF THE INVENTION

### 1. Field of the Invention

This invention relates to the field of mycotoxin assays. More particularly, this invention relates to a homogeneous assay that uses changes in fluorescence polarization to detect the presence of deoxynivalenol in grains.

### 2. Description of Related Art

Deoxynivalenol (DON), which is also known as vomitoxin, is a mycotoxin produced in various grains, such as wheat, corn, barley, oats and rye, by *Fusarium graminearum* and other *Fusarium* strains¹⁻⁴. Presence of DON in food causes food refusal, vomiting and growth depression in swine, gastrointestinal illness in humans, and embryotoxicity and immunotoxicity in laboratory animals¹⁻⁴. Because of its latent health risks, research is being carried out to explore analytical methods of detecting DON.

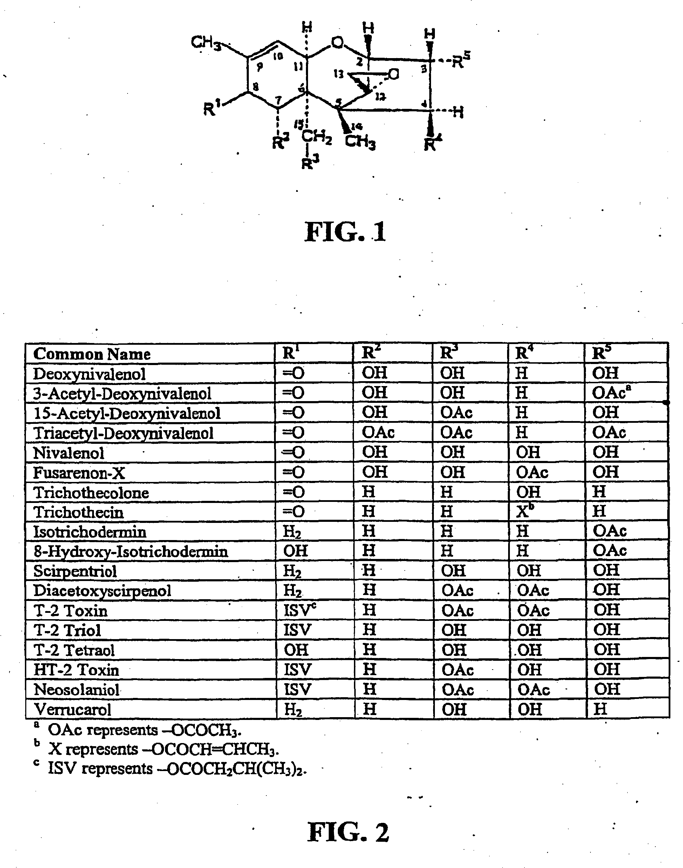

More generally, DON is a particularly troublesome mycotoxin within the group of mycotoxins known as trichothecenes. As shown in Figure 1, trichothecenes have a common skeleton that can have different groups attached at R¹-R⁵. Figure 2 identifies the R¹-R⁵ groups in Figure 1 for DON and for some of the other more well-known trichothecenes.

Various methods for the quantitative analysis of DON and other trichothecenes in grains are in use, such as thin-layer chromatography (TLC), gas chromatography (GC), and high-pressure liquid chromatography (HPLC). The TLC method is an official method (first action) of AOAC International. Although relatively simple, the TLC method lacks sensitivity and is only semiquarititative. In addition, most of these chromatographic methods require extensive cleanup procedures after extraction and are not suitable for field testing^{4,5}.

Recently, enzyme-linked immunosorbent assay (ELISA) methods have been successfully applied to the screening of DON in grains². However, ELISA methods are undesirably labor intensive, in that they typically involve several washings, liquid transfers, and incubation times. Accordingly, there is a need for a simple, yet sensitive, method for the determination of DON and other trichothecenes in grains that is rapid and field portable.

In Nielsen, K. et al., "A homogeneous fluorescence polarization assay for detection of antibody to Brucella abortus", Journal of Immunological Methods, 1996, 195, 161-168, a homogeneous fluorescence polarization assay for detection of antibody to *Brucella abartus* in bovine sera is described. In Ligler, F.S. et al., "A Homogeneous Immunoassay for the Mycotoxin T-2 Utilizing Liposomes, Monoclonal Antibodies, and Complcment", Analytical Biochemishy, 1987, 163, 369-375, a homogeneous competition inhibition assay for the trichothecene mycotoxin T-2 based on complement-mediated lysis of liposomes is described. In Adamczyk, M. et al., "Synthesis of Conjugates for a Barbiturate Screening Assay", Bioconjugate Chem., 1997, 8, 281-288 derivatives of barbiturates and their use for developing a fluorescence polarization immunoassay are described.

### SUMMARY OF THE INVENTION

In a first principal aspect, the present invention provides a tracer for use in a homogeneous assay for the determination of deoxynivalenol (DON) in grains, the tracer comprising a fluorophore conjugated to DON, wherein the tracer is able to bind to an antibody specific to DON to produce a detectable change in fluorescence polarization.

In a second principal aspect, the present invention provides a homogeneous assay for the determination of trichothecenes in grains. In accordance with the method, trichothecene is extracted from a grain sample. The extract is combined with a tracer and an antibody to provide a mixture. The tracer comprises a predetermined trichothecene conjugated to a fluorophore, and the tracer is able to bind to the antibody to produce a detectable change in fluorescence polarization. The fluorescence polarization of the mixture is then measured. The measured fluorescence polarization is compared with a characterized fluorescence polarization value that corresponds to a known trichothecene concentration.

### BRIEF DESCRIPTION OF THE DRAWINGS

Figure 1 shows the basic chemical structure of trichothecenes, with R¹, R², R³, R⁴, and R⁵ representing substitution positions on the basic trichothecene skeleton.
Figure 2 is a table identifying the R¹-R⁵ groups in Figure 1 for some well-known trichothecenes.
Figure 3 is a standard curve for a fluorescence polarization assay for DON, using the data of Table 1, in accordance with a preferred embodiment of the present invention.
Figure 4 is a standard curve for a fluorescence polarization assay for DON, using the data of Table 2, in accordance with a preferred embodiment of the present invention.
Figure 5 is a standard curve for a fluorescence polarization assay for DON, using the data of Table 3, in accordance with a preferred embodiment of the present invention.
Figure 6 is a graph comparing the DON concentration of spiked samples with the DON concentration calculated from the standard curve of Figure 5, in accordance with a preferred embodiment of the present invention.
Figure 7 is a standard curve for a fluorescence polarization assay for DON, using the data of Table 5, in accordance with a preferred embodiment of the present invention.
Figure 8 is a graph comparing the DON concentration of samples as measured using HPLC with the DON concentration as calculated from the standard curve of Figure 7, in accordance with a preferred embodiment of the present invention.

### DETAILED DESCRIPTION OF THE PREFERRED EMBODIMENTS

The present invention describes a homogeneous assay for the determination of trichothecenes such as DON in grains that is based on measurements of fluorescence polarization. The technique of fluorescence polarization has been successfully utilized in various assays involving proteins, enzymes, therapeutic drugs, drugs of abuse, DNA, hormones, peptides and antibodies⁶⁻⁸.

The principle behind the fluorescence polarization technique is as follows. Fluorescent probes having low molecular weight have low polarization values due to their fast rotation, whereas fluorescent probes with higher molecular weight have higher polarization due to their slower rotation. Thus the polarization value of a fluorophore increases upon binding to a larger molecule. Further information about the fluorescence polarization technique is provided in U.S. Patent Nos. 5,427,960 and 5,976,820 and in Nasir, M. S. and Jolley, M. E., "Fluorescence Polarization: An analytical tool for Immunoassay and Drug Discovery," Combinatorial Chemistry & High Throughput Screening, 1999, 2, 177-190.

The assay uses a tracer, comprising a fluorophore conjugated to DON, that provides a specified polarization value and is able to bind to an antibody specific to DON to produce a detectable change in fluorescence polarization. This DON-fluorophore tracer competes with free DON from grain extracts for binding to a specific antibody (an inhibition assay), giving rise to a fluorescence polarization value that is inversely proportional to the quantity of DON in the grain.

In preferred embodiments, the assay is rapid, simple (in that it requires minimal training), and field portable, making it useful for the routine and quantitative determination of DON in grains. The preferred assay can also detect very small amounts of DON (<0.2 PPM).

### 1. Materials and Methods

All the chemicals and solvents were used as received unless otherwise noted. DON and fluoresceinamine (isomer II) were obtained from Sigma. Monoclonal antibodies specific to DON were provided by Dr. Chris Maragos (USDA, Peoria, IL). Reaction mixtures were separated on preparative TLC using silica gel plates (Sigma). Fluorescence polarization was measured at room temperature using a single tube Sentry-FP fluorescence polarization instrument (Diachemix Corp.). Data was plotted using the program Graph Pad Prism, and unknown values were obtained from the graph.

### 2. DON Monoclonal Antibody Preparation

A number of different techniques for preparing DON specific antibodies have been reported by various groups^{1-2,9}. Since DON is a small molecule, it is typically conjugated with a protein to be made immunogenic.

The preferred method for preparing DON specific antibodies, which was the method used to prepare the antibodies used in these studies, is as follows. DON (5 mg) is reacted with 1,1'-carbonyldiimidazole (40 mg) in 800 µl acetone for 1 hour at room temperature. Water (20 µl) is slowly added followed by 5.8 mg of ovalbumin in 385 µl of 0.1 M sodium bicarbonate buffer (pH 8.5). The mixture is kept at 4° C for 24 hours and dialyzed extensively against 0.1 M phosphate-buffered saline (PBS). The DON-protein conjugate is diluted to 1 mg/ml with 0.1 M PBS, distributed as 200 µl portions, and lyophilized. This lyophilized material is reconstituted with water immediately before using it to immunize mice. The antibodies produced by these cell lines are then screened for DON specificity. Further details are provided in Chris M. Maragos and Susan P. McCornnick, "Monoclonal Antibodies for the Mycotoxins Deoxynivalenol and 3-Acetyl-Deoxynivalenol," Food and Agricultural Immunology, 2000, 12, 181-192.

### 3. Preparation of DON-F Tracer

100 µL of an acetone solution of DON (Sigma, 0.625 mg) was mixed with 2 mg of 1,1 carbonyldiimidazole (Sigma) and kept at room temperature. After two hours, this was mixed with 100 µl of a solution of fluoresceinamine isomer II (6-aminofluorescein, Sigma, 10 mg/ml in 0.1 molar sodium carbonate, pH ~ 9.5) and after thorough shaking, the reaction was incubated overnight at room temperature. The resultant product was separated by preparative TLC (silica, CHCl₃: CH₃OH: CH₃CO₂H, 90: 10: 1), and the product (Rf ~ 0.4) was collected, shaken with methanol, centrifuged and filtered to give pure DON-F tracer. This stock solution of DON-F tracer was stored at 2-8° C and diluted appropriately for use. Other fluorophores could also be used, depending on the antibody.

It was observed that 10 µl of this diluted tracer in 1 ml PBS gave an intensity equivalent to ~ 1 nM fluorescein. Furthermore, this tracer was found to give a polarization of 40-50 mP, which changed to ~250 mP upon adding the appropriately diluted DON antibody provided by Dr. Chris Maragos (USDA, Peoria, IL).

### 4. Protocol for DON Fluorescence Polarization Assay

A preferred protocol for performing the DON assay is as follows. The grain sample being tested for DON is crushed. Then, 20 grams of the crushed grain is shaken in 100 ml of water for 10-20 minutes to extract the DON for analysis. Beneficially, this extract may be used without any additional purification steps.

In order to determine the amount of DON present in the grain samples, a standard curve is first obtained using standard DON solutions as follows. A series of standard DON solutions may be made by diluting an appropriate amount of stock aqueous DON solution (10 mg/ml) into 1 m1 PBSA buffer that contains bovine gamma globulin (BGG) in a concentration of 0.01%. A fixed amount of standard, such as 10 µl or 20 µl, is pipetted into 1 ml of diluted antibody solution in a 10x75 mm glass test tube, and the mixture is vortexed thoroughly. The diluted antibody solution is preferably about 1/20,000 to 1/40,000 in PBSA with BGG. This mixture is used to perform a blank reading in the instrument. The readings should be repeated until they are stable (normally two readings). 10 µl of DON-F tracer solution, prepared as described above, is then added to the glass test tube containing the antibody solution and standard, and the test tube is vortexed thoroughly. The test tube is placed back in the instrument, and mP values are recorded until they stabilize. The other standard solutions are read in the same way, and a standard curve is constructed using the stabilized mP values.

The samples, prepared as described above, are measured in a similar manner. Specifically, a fixed amount of extracted sample, typically 10 µl or 20 µl, depending on what was used for the standard curve, is pipetted into 1 ml of diluted antibody solution in a 10x75 mm glass test tube. The mixture is vortexed thoroughly and used to perform a blank reading in the instrument. 10 µl of tracer is added to the glass test tube containing the antibody solution and sample, and the test tube is vortexed thoroughly. The test tube is placed back in the instrument, and mP values are recorded. The DON concentration in the extracted sample is then calculated using the standard curve.

### 5. Standard Curve For Fluorescence Polarization Assay of DON

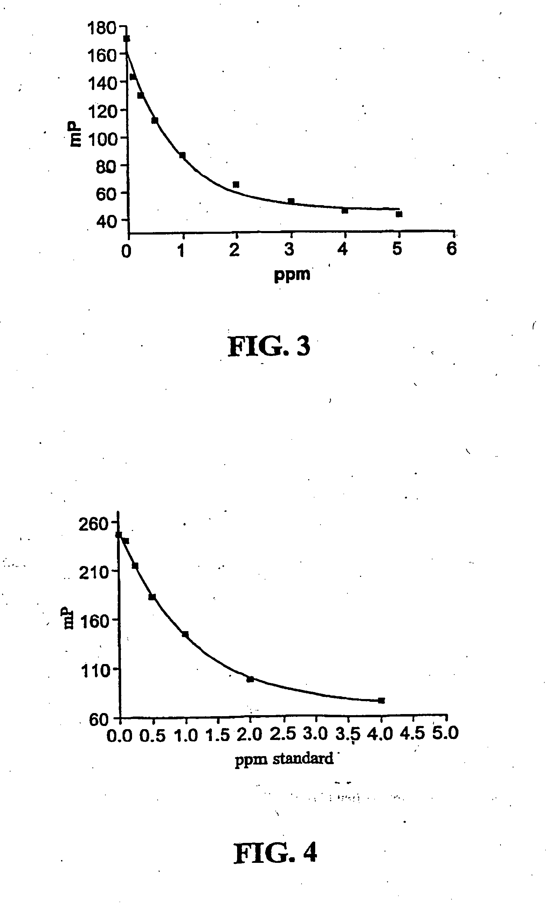

Standard curves were obtained for the Sentry-FP fluorescence polarization instrument using the protocol described above for DON standards in various concentrations. Table 1 lists the fluorescence polarization readings in mP units for DON standard solutions, using 10 µl of standard solution and "Antibody #1" from Dr. Chris Maragos (USDA, Peoria, IL). Figure 3 shows the standard curve, relating the fluorescence polarization signal in mP to DON concentration in ppm, that was obtained from this data.

**TABLE 1**

| **DON standard concentration (ppm)** | **mP** |
|---|---|
| 0.00 | 171 |
| 0.10 | 143 |
| 0.25 | 130 |
| 0.50 | 112 |
| 1.00 | 87 |
| 2.00 | 65 |
| 3.00 | 52 |
| 4.00 | 46 |
| 5.00 | 43 |

Table 2 lists the fluorescence polarization readings in mP units for DON standard solutions, using 20 µl of standard solution and "Antibody #4" from Dr. Chris Maragos (USDA, Peoria, IL). Figure 4 shows the standard curve, relating the fluorescence polarization signal in mP to DON concentration in ppm, that was obtained from this data.

**TABLE 2**

| **DON standard concentration (ppm)** | **mP** |
|---|---|
| 0.00 | 247 |
| 0.10 | 241 |
| 0.25 | 215 |
| 0.50 | 183 |
| 1.00 | 145 |
| 2.00 | 98 |
| 4.00 | 76 |

### 6. Results of Fluorescence Polarization Assay For DON Spiked Wheat Samples

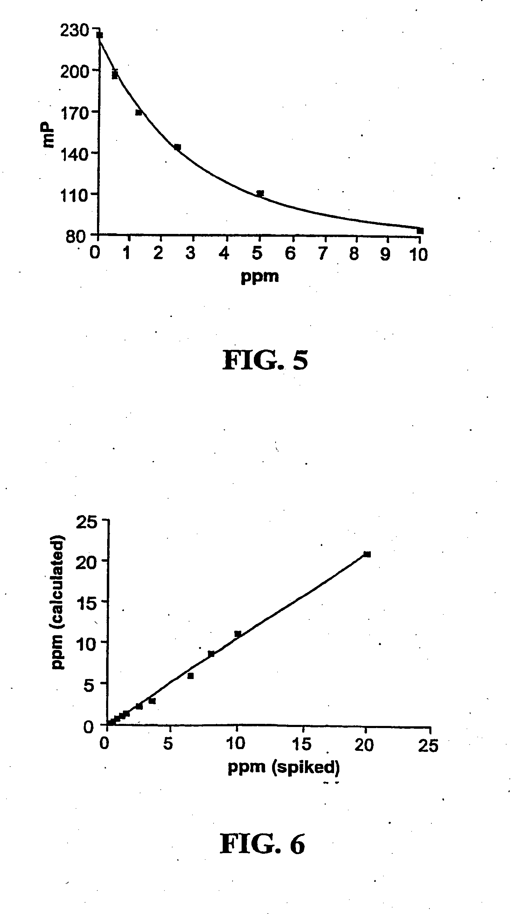

DON free wheat was supplied by Dr. Chris Maragos (USDA, Peoria, IL). 20 g wheat samples were supplemented with varying concentrations of DON (Sigma, 1 mg/ml stock solution) and extracted with 100 ml water by shaking for 10-20 minutes. 1 ml of the supernatant was centrifuged in each case and analyzed. If not analyzed immediately, samples were stored in a refrigerator. 20 µl of sample was mixed with 1 ml of a 1/40,000 solution of "Antibody #1" from Dr. Chris Maragos (USDA, Peoria, IL) in PBSA-BGG buffer and blanked in the Sentry-FP instrument. 10 µl of properly diluted tracer was then added and the fluorescence polarization measured. Each sample was run in duplicate. A standard graph was plotted (Figure 5), using the data of Table 3. "Antibody #4," however, was found not to yield reliable results with real samples.

**TABLE 3**

| **DON standard concentration (ppm)** | **mP (run 1)** | **mP (run 2)** | **mP (run 3)** |
|---|---|---|---|
| 0 | 224 | 228 | 223.45 |
| 0.5 | 203.08 | 192.33 | 196 |
| 1.25 | 168.89 | 171.49 | 169.02 |
| 2.5 | 143.2 | 145 | 146.42 |
| 5 | 111.12 | 110.07 | 112.01 |
| 10 | 82.68 | 84.92 | 86.52 |

The DON concentration (in ppm) of each sample was calculated from the standard graph of Figure 5, using the average mP value of the duplicate runs of that sample. Table 4 shows these results. Figure 6 is a plot of the DON concentration (in ppm) of the samples as they were spiked versus the DON concentration (in ppm) calculated from the standard curve of Figure 5. A good correlation (an r² value of 0.994) was observed between these theoretical and calculated DON concentrations.

**TABLE 4**

| **DON concentration of spiked sample (ppm)** | **mP** | **Calculated DON concentration (ppm)** | **% Recovery of DON** |
|---|---|---|---|
| 3.5 | 134.03 | 2.95 | 84.28 |
| 0.5 | 200.52 | 0.49 | 98 |
| 1.5 | 169.00 | 1.42 | 94.66 |
| 6.5 | 100.64 | 6.00 | 92.3 |
| 10 | 178.29 | 11.1 | 111 |
| 20 | 151.03 | 21 | 105 |
| 0.2 | 209.85 | 0.26 | 130 |
| 1.2 | 177.30 | 1.14 | 95 |
| 0.8 | 188.01 | 0.82 | 102 |
| 2.5 | 147.17 | 2.28 | 91.2 |
| 8 | 186.31 | 8.7 | 108 |

### 7. Comparison of Fluorescence Polarization Assay For DON With HPLC

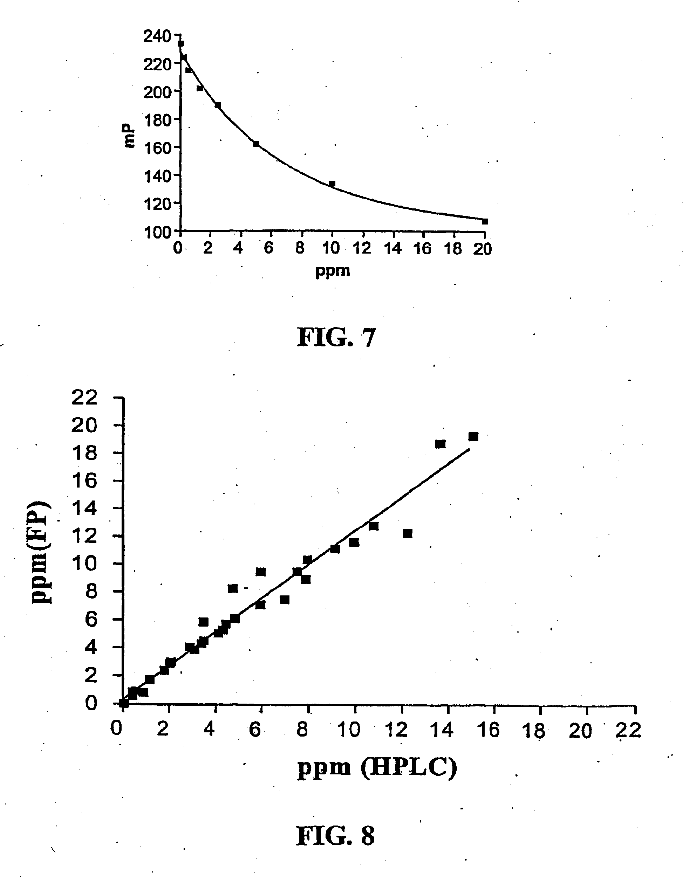

A number of different wheat samples with varying amounts of DON, provided by Dr. Chris Maragos (USDA, Peoria, IL), were analyzed by the fluorescence polarization assay procedure described above using Antibody #1. The samples were also analyzed by HPLC using standard methods. Table 5 shows the data for the standard graph (Figure 7) used in this study.

**TABLE 5**

| **DON standard concentration (ppm)** | **mP (run 1)** | **mP (run 2)** | **mP (run 3)** | **mp (run 4)** |
|---|---|---|---|---|
| 0 | 235 | 235 | 234 | 232 |
| 0.2 | 225 | 228 | 222 | 222 |
| 0.5 | 214 | 217 | 215 | 214 |
| 1.25 | 201 | 201 | 203 | 203 |
| 2.5 | 190 | 190 | 191 | 191 |
| 5 | 161 | 161 | 164 | 163 |
| 10 | 134 | 135 | 133 | 133 |
| 20 | 107 | 107 | 108 | 109 |

Table 6 shows the DON concentration (in ppm) of each sample calculated from the standard graph of Figure 7.

**TABLE 6**

| **Sample Number** | **mP** | **Calculated DON Concentration (ppm)** |
|---|---|---|
| 1 | 133.5 | 9.46 |
| 2 (1x10 dilution) | 196.75 | 1.93 |
| 3 | 182.25 | 3.06 |
| 4 | 139.5 | 8.26 |
| 5 | 144 | 7.48 |
| 6 | 125 | 11.64 |
| 7 | 123 | 12.27 |
| 8 | 136 | 8.93 |
| 9 | 110.5 | 18.73 |
| 10 | 153.25 | 6.12 |
| 11 | 133.5 | 9.46 |
| 12 | 156.25 | 5.73 |
| 13 | 228.5 | 0.04 |
| 14 | 234 | 0.00 |
| 15 | 121.5 | 12.79 |
| 16 | 126.75 | 11.13 |
| 17 | 129.75 | 10.33 |
| 18 | 146.25 | 7.12 |
| 19 | 166.5 | 4.55 |
| 20 | 173 | 3.89 |
| 21 | 190 | 2.43 |
| 22 | 159.75 | 5.30 |
| 23 | 168.5 | 4.34 |
| 24 | 237.75 | 0.00 |
| 25 | 170.75 | 4.11 |
| 26 | 199 | 1.77 |
| 27 | 213.5 | 0.82 |
| 28 | 217.5 | 0.58 |
| 29 | 212 | 0.92 |
| 30 | 155 | 5.89 |
| 31 | 213.25 | 0.84 |
| 32 | 161.75 | 5.07 |
| 33 | 228 | 0.05 |
| 34 | 184.25 | 2.89 |

Figure 8 is a plot of the calculated DON concentrations (in ppm) from the fluorescence polarization assay, as shown in Table 6, versus the DON concentration (in ppm) of the sample as measured by HPLC. A best fit line was calculated from this data. Its slope was 1.2112 ± 0.0391, and the r² value was 0.9676, indicating that the results from the fluorescence polarization assay were in good agreement with the results from HPLC.

### 8. Other Trichothecenes

It has been found that Antibody #1, when used in a fluorescence polarization assay with the DON-F tracer as described above, has some cross-reactivity with other trichothecenes. Specifically, Antibody #1 was found to have a 358% cross-reactivity with 15-acetyl-deoxynivalenol and a 9% cross-reactivity with HT-2 toxin. The other trichothecenes listed in Figure 2 were found to have less than a 4% cross-reactivity.

The cross-reactivity percentage for each trichothecene was calculated by dividing the IC₅₀ value for DON by the IC₅₀ value of the trichothecene and multiplying by 100%. The IC₅₀ value of a trichothecene is the concentration of the trichothecene required to give a fluorescence polarization response of 50%, where 100% corresponds to the response of the assay without the trichothecene and 0% represents the response of the assay without antibody.

Because of this cross-reactivity, the fluorescence polarization assay described herein may be used for the determination of trichothecenes other than DON. Moreover, because DON is closely related to other trichothecenes, fluorescence polarization assays may be developed specifically for these other trichothecenes.

### 9. Assay Kit

The materials used to perform the assay of the present invention are preferably made available in kit form. The kit preferably includes tracer and antibody in an amount suitable for at least one assay, along with suitable packaging and instructions for use. The tracer and antibody may be provided in solution, as a liquid dispersion, or as a substantially dry powder (e.g., in lyophilized form).

The suitable packaging can be any solid matrix or material, such as glass, plastic, paper, foil, and the like, capable of separately holding within fixed limits the buffer, tracer, and antibody. For example the tracer and monoclonal antibody may be provided as solutions in separate labeled bottles or vials made of glass or plastic.

For the determination of DON, the antibody is specific for DON and is preferably a monoclonal antibody. The preferred monoclonal antibody may be prepared as described herein and as known in the art.

For the determination of DON, the tracer comprises a fluorophore, such as 6-aminofluorescein, conjugated to DON. Other fluorophores may be used, provided the resulting tracer is able to bind with the monoclonal antibodies to produce a detectable change in fluorescence polarization.

### 10. References

1: Sinha, R. C.; Savard, M. E.; Lau, R. "Production of Monoclonal Antibodies for the Specific Detection of Deoxynivalenol and 15-Acetyldeoxynivalenol by ELISA," J. Agric. Food Chem, 1995, 43, 1740-1744.
2: ((a) Casale, W. L.; Pestka, J. J.; Hart, L. P. "Enzyme-Linked Immunosorbent Assay Employing Monoclonal Antibody Specific for Deoxynivalenol (Vomitoxin) and Several Analogues," J. Agric. Food Chem, 1988, 36, 663-668. (b) Pestka, J. J.; Abouzied, M. N.; Sutikno. "Immunological Assays for Mycotoxin detection," Food Technology, 1995, 120-128.
3: Trucksess, M. W.; Ready, D. E.; Pender, M. K.; Ligmond, C. A.; Wood, G. E.; Page, S. W. "Determination and Survey of Deoxynivalenol in White Flour, Whole Wheat Flour and Bran," J. AOAC Int., 1996, 79, 883-887.
4: Trucksess, M. W.; Nesheim, S.; Eppley, R. M. "Thin Layer Chromatographic Determination of Deoxynivalenol in Wheat and Corn," J. Assoc. Off. Anal. Chem., 1984, 67, 40-43.
5: Chang, H. L.; DeVries, J. W.; Larson, P. A.; Patel, H. H. "Rapid Determination of DON cleanup," JAOAC, 1984, 67, 52-57.
6: Nielsen, K.; Gall, D.; Jolley, M.; Leishman, G.; Balsevicius, S.; Smith, P.; Nicoletti, P.; Thomas, F,. J. Immun. Methods, 1996, 195, 161-168.
7: ((a) Lynch, B. A.; Loiacono, K. A.; Tiong, C. L.; Adams, A. E.; MacNeil, I. A. "A fluorescence polarization based Src-SH2 binding assay," Anal. Biochem., 1997, 247, 77-82. (b) Wei, A. P.; Herron, J. N., Anal. Chem.. 1993, 65, 3372-3377. (c) Kauvar, L. M.; Higgins, D. L.; Viller, H. O.; Sportsman, J. R.; Engquist-Goldstein, A.; Bukar, R.; Bauer, K. E.; Dilley, H.; Rocke, D. M., Chem. Biol., 1995, 2, 107-118.
8: ((a) Nasir, M. S.; Jolley, M. E. "Fluorescence Polarization: An Analytical Tool for Immunoassays and Drug Discovery," Combinatorial Chem. & High Throughput Screening, 1999, 2, 177-190. (b) Jolley, M. E. "Fluorescence polarization immunoassay for the determination of therapeutic drug levels in human plasma," J. Anal. Toxicol., 1981, 5, 236-240. (c) Eremin, S. A.; Gallacher, G.; Lotey, H.; Smith, D. S.; Landon, J., Clin. Chem., 1987, 33, 4113-4122. (d) Jolley, M. E., "Fluorescence polarization assays for the detection of proteases and their inhibitors," J. Biomol. Screen., 1996, 1, 33-38
9: Zhang, G.; Li, S. W.; Chu, F. S., "Production and characterization of antibody against deoxynivalenol triacetate," J. Food Protection, 1986, 49, 336-339.

## Claims

1. A tracer for use in a homogeneous assay for the determination of deoxynivalenol (DON) in grains, the tracer comprising a fluorophore conjugated to DON, wherein the tracer is able to bind to an antibody specific to DON to produce a detectable change in fluorescence polarization.

2. The tracer of claim 1, wherein the fluorophore is 6-aminofluorescein.

3. The tracer of claim 1, wherein the antibody specific to DON is a monoclonal antibody.

4. A homogeneous assay for the determination of trichothecenes in grains, said homogeneous assay comprising the steps of:
extracting trichothecene from a grain sample to provide an extract;
combining said extract with a tracer and an antibody to provide a mixture, said tracer comprising a predetermined trichothecene conjugated to a fluorophore, said tracer being able to bind to said antibody to produce a detectable change in fluorescence polarization;
measuring the fluorescence polarization of said mixture to obtain a measured fluorescence polarization; and
comparing said measured fluorescence polarization with a characterized fluorescence polarization value, said characterized fluorescence polarization value corresponding to a known trichothecene concentration.

5. The assay of claim 4, wherein said predetermined trichothecene is deoxynivalenol (DON).

6. The assay of claim 5, wherein said fluorophore is 6-aminofluorescein.

7. The assay of claim 4, further comprising the steps of:
providing a plurality of trichothecene standard solutions, each of said standard trichothecene solutions having a different known concentration of trichothecene;
adding said tracer and said antibody to each one of said plurality of trichothecene standard solutions, so as to provide a plurality of standard mixtures; and
measuring the fluorescence polarization of each one of said plurality of said standard mixtures to provide a plurality of standard fluorescence polarization values corresponding to known trichothecene concentrations.

8. The assay of claim 7, wherein said characterized fluorescence polarization value is one of said standard fluorescence polarization values.

## Patentansprüche

1. Tracer zur Verwendung in einem homogenen Test für die Bestimmung von Desoxynivalenol (DON) in Getreide, wobei der Tracer ein Fluorophor in Konjugation an DON umfasst und fähig ist, an einen DON-spezifischen Antikörper zu binden, um eine nachweisbare Veränderung der Fluoreszenzpolarisation zu erzeugen.

2. Tracer nach Anspruch 1, wobei das Fluorophor 6-Aminofluorescein ist.

3. Tracer nach Anspruch 1, wobei der DON-spezifische Antikörper ein monoklonaler Antikörper ist.

4. Homogener Test für die Bestimmung von Trichothecenen in Getreide, wobei der homogene Test die Schritte umfasst:
Extrahieren von Trichothecen aus einer Getreideprobe, um einen Extrakt bereitzustellen,
Kombinieren des Extrakts mit einem Tracer und einem Antikörper, um ein Gemisch bereitzustellen, wobei der Tracer ein vorbestimmtes Trichothecen in Konjugation an ein Fluorophor umfasst und fähig ist, an den Antikörper zu binden, um eine nachweisbare Veränderung der Fluoreszenzpolarisation zu erzeugen,
Messen der Fluoreszenzpolarisation des Gemisches, um eine gemessene Fluoreszenzpolarisation zu erhalten, und
Vergleichen der gemessenen Fluoreszenzpolarisation mit einem charakterisierten Fluoreszenzpolarisationswert, wobei der charakterisierte Fluoreszenzpolarisationswert einer bekannten Trichothecen-Konzentration entspricht.

5. Test nach Anspruch 4, wobei das vorbestimmte Trichothecen Desoxynivalenol (DON) ist.

6. Test nach Anspruch 5, wobei das Fluorophor 6-Aminofluorescein ist.

7. Test nach Anspruch 4, der ferner die Schritte umfasst:
Bereitstellen mehrerer Trichothecen-Standardlösungen, wobei eine jede der Trichothecen-Standardlösungen eine unterschiedliche bekannte Konzentration an Trichothecen aufweist,
Hinzufügen des Tracers und des Antikörpers zu jeder der mehreren Trichothecen-Standardlösungen, um mehrere Standardgemische bereitzustellen, und
Messen der Fluoreszenzpolarisation eines jeden der mehreren Standardgemische, um mehrere Standardfluoreszenzpolarisationswerte, die bekannten Trichothecen-Konzentrationen entsprechen, bereitzustellen.

8. Test nach Anspruch 7, wobei der charakterisierte Fluoreszenzpolarisationswert ein Wert der Standardffuoreszenzpolarisationswerte ist.

## Revendications

1. Traceur pour utilisation dans une analyse homogène pour la détermination de désoxynivalénol (DON) en grains, le traceur comprenant un fluorophore conjugué au DON, tandis que le traceur est apte à se lier à un anticorps spécifique pour le DON pour produire un changement détectable de la polarisation de fluorescence.

2. Traceur selon la revendication 1, dans lequel le fluorophore est la 6-aminofluorescéine.

3. Traceur selon la revendication 1, dans lequel l'anticorps spécifique pour le DON est un anticorps monoclonal.

4. Analyse homogène pour la détermination de trichothécènes dans des grains, ladite analyse homogène comprenant les étapes de:
extraction de trichothécène d'un échantillon de grains pour produire un extrait;
combinaison dudit extrait avec un traceur et un anticorps pour produire un mélange, ledit traceur comprenant un trichothécène prédéterminé conjugué à un fluorophore, ledit traceur étant apte à se lier audit anticorps pour produire un changement détectable dans la polarisation de fluorescence;
mesure de la polarisation de fluorescence dudit mélange pour obtenir une polarisation de fluorescence mesurée; et
comparaison de ladite polarisation de fluorescence mesurée avec une valeur de polarisation de fluorescence caractérisée, ladite valeur de polarisation de fluorescence caractérisée correspondant à une concentration de trichothécène connue.

5. Analyse selon la revendication 4, dans laquelle ledit trichothécène est le désoxynivalénol (DON).

6. Analyse selon la revendication 5, dans laquelle ledit fluorophore est la 6-aminofluorescéine.

7. Analyse selon la revendication 4, comprenant en outre les étapes de:
fourniture d'une pluralité de solutions étalon de tricliothécène, chacune desdites solutions de trichothécène étalon ayant une concentration connue différente de trichothécène;
addition dudit traceur et dudit anticorps à chacune parmi ladite pluralité de solutions étalon de trichothécène, de manière à produire une pluralité de mélanges étalon; et
mesure de la polarisation de fluorescence de chacun parmi ladite pluralité desdits mélanges étalon pour produire une pluralité de valeurs de polarisation de fluorescence étalon correspondant à des concentrations de trichothécène connues.

8. Analyse selon la revendication 7, dans laquelle ladite valeur de polarisation de fluorescence caractérisée est l'une desdites valeurs étalon de polarisation de fluorescence.
